# EUROPEAN PATENT SPECIFICATION

(11) **EP 4 613 178 B1**
(45) Date of publication and mention of the grant of the patent: **22.07.2026**
(21) Application number: 23925977.3
(22) Date of filing: 08.10.2023
(51) Int. Cl.: A61B 5/00, G02B 21/00, G02B 27/58

(54) **SUPER-RESOLUTION IMAGING METHOD AND APPARATUS BASED ON LINE SCAN**
HOCHAUFLÖSENDES BILDGEBUNGSVERFAHREN UND VORRICHTUNG AUF BASIS VON ZEILENABTASTUNG
PROCÉDÉ ET APPAREIL D'IMAGERIE À SUPER-RÉSOLUTION BASÉS SUR UN BALAYAGE DE LIGNE

(30) Priority: 06.03.2023 CN 202310207189
(43) Date of publication of application: 10.09.2025
(73) Proprietor: Center for Excellence in Brain Science and Intelligence Technology, Chinese Academy of Sciences, Xuhui District Shanghai 200031 (CN)
(72) Inventor: WANG, Kai, Shanghai 200031 (CN); ZHANG, Yujie, Shanghai 200031 (CN); CONG, Lin, Shanghai 200031 (CN); YE, Lichen, Shanghai 200031 (CN)
(74) Representative: Hauck Patent- und Rechtsanwälte PartmbB
(86) International application number: PCT/CN2023/123332
(87) International publication number: WO 2024/183279

(56) References cited:
- CN-A- 106 980 174
- CN-A- 107 015 353
- CN-A- 110 954 520
- CN-A- 114 565 514
- CN-A- 116 195 977
- US-A1- 2019 227 293
- US-A1- 2020 103 639
- US-A1- 2022 343 463
- ZIWEI LI ET AL: "Contrast and resolution enhanced optical sectioning in scattering tissue using line-scanning two-photon structured illumination microscopy", OPTICS EXPRESS, vol. 25, no. 25, 11 December 2017 (2017-12-11), pages 32010, XP055648938, DOI: 10.1364/OE.25.032010
- PHILIPP J KELLER ET AL: "Fast, high-contrast imaging of animal development with scanned light sheet-based structured-illumination microscopy", NATURE METHODS, vol. 7, no. 8, 1 August 2010 (2010-08-01), New York, pages 637 - 642, XP055558226, ISSN: 1548-7091, DOI: 10.1038/nmeth.1476
- THRAPP ANDREW D. ET AL: "Reduced motion artifacts and speed improvements in enhanced line-scanning fiber bundle endomicroscopy", JOURNAL OF BIOMEDICAL OPTICS, vol. 26, no. 05, 13 May 2021 (2021-05-13), 1000 20th St. Bellingham WA 98225-6705 USA, XP093340152, ISSN: 1083-3668, DOI: 10.1117/1.JBO.26.5.056501

## Description

### CROSS-REFERENCE TO RELATED APPLICATIONS

This application is a national stage of International Application No. PCT/CN2023/123332, filed on October 08, 2023, which claims priority to Chinese Patent Application No. 202310207189.0 filed on March 6, 2023.

### TECHNICAL FIELD

The present disclosure generally relates to the field of imaging technology, and more particular, to a super-resolution imaging method and a super-resolution imaging apparatus based on line scan.

### BACKGROUND

Traditional imaging technique such as confocal imaging technique, wide-field imaging technique, etc. is limited by the Abbe diffraction limit, so that its maximum imaging resolution is typically around 250 nanometers (nm), making it incapable of resolving structures with smaller size. In view of this, super-resolution imaging technology came into being, which can exceed the limitation of the diffraction limit, thereby enabling the imaging resolution to reach 100 nm or even smaller, providing critical technical support for researches on micro-size structures.

"Contrast and resolution enhanced optical sectioning in scattering tissue using line-scanning two-photon structured illumination microscopy" (by Ziwei Li et al., OPTICS EXPRESS, vol. 25, no. 25, 11 December 2017, page 32010, DOI: 10.1364/OE.25.032010) discloses an optical sectioning enhanced two-photon technique which incorporates structured illumination into line-scanning spatial-temporal focusing microscopy (LTSIM), and generate patterned illumination via laser intensity modulation synchronized with scanning. LTSIM brings scattering background elimination and in-focus contrast enhancement, and realizes nearly 2-fold increase in spatial resolution to ~208 nm laterally and ~0.94 µm axially. In addition, the intensity modulated line-scanning implementation of LTSIM enables fast and flexible generation of structured illumination, permitting adjustable spatial frequency profiles to optimize image contrast.

### SUMMARY

According to an aspect of the present disclosure, a super-resolution imaging method based on line scan is provided, including: providing multiple types of structured line excitation light, each of the multiple types of structured line excitation light having a different illumination mode; illuminating a target area in a manner of row-by-row scanning along a single first direction while switching the multiple types of structured line excitation light, such that each row in the target area is illuminated by a corresponding type of structured line excitation light among the multiple types of structured line excitation light; and detecting response light generated by the each row in the target area in response to being illuminated by the corresponding type of structured line excitation light.

According to another aspect of the present disclosure, a super-resolution imaging apparatus based on line scan is provided, including: an illumination module configured to provide multiple types of structured line excitation light, each of the multiple types of structured line excitation light having a different illumination mode; a scanning module configured to illuminate a target area in a manner of row-by-row scanning along a single first direction while switching the multiple types of structured line excitation light, such that each row in the target area is illuminated by a corresponding type of structured line excitation light among the multiple types of structured line excitation light; and a detection module configured to detect response light generated by the each row in the target area in response to being illuminated by the corresponding type of structured line excitation light.

Other features of the present disclosure and advantages thereof will become more apparent from the following detailed description of exemplary embodiments of the present disclosure with reference to the accompanying drawings.

### BRIEF DESCRIPTION OF THE DRAWINGS

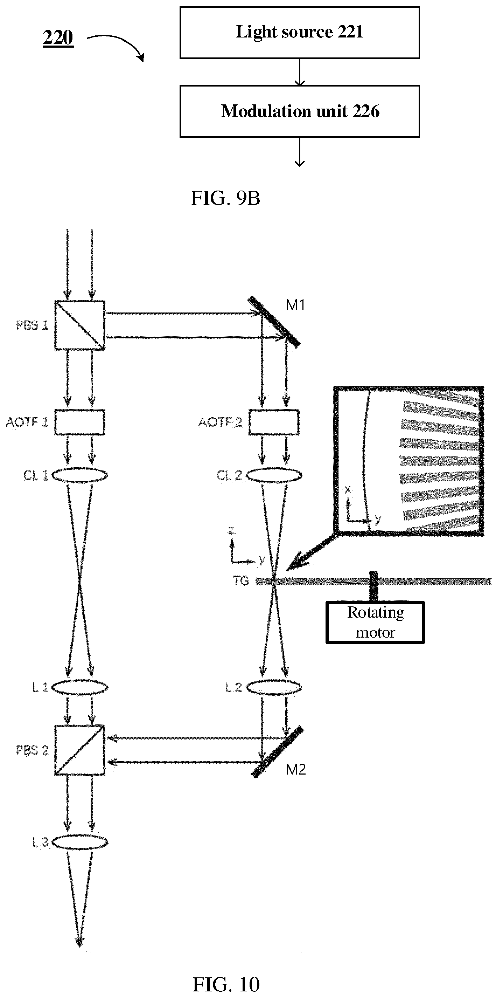

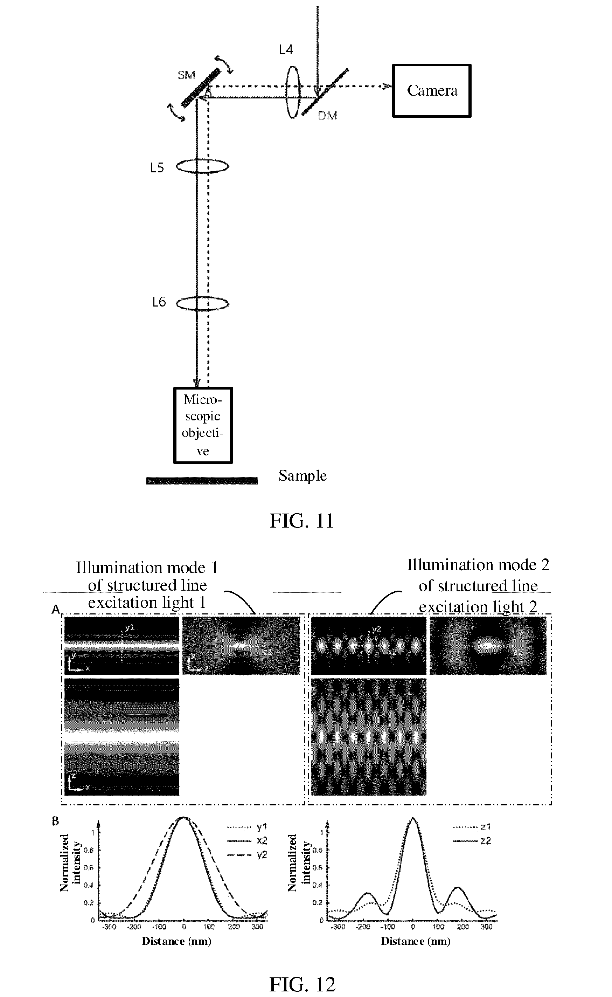

From the following description of embodiments of the present disclosure shown in conjunction with the drawings, the foregoing and other features and advantages of the present disclosure will become clear. The drawings are incorporated herein and form a part of the specification, which are further used to explain the principles of the present disclosure and enable those skilled in the art to make and use the present disclosure, in which:
FIG. 1A is a schematic diagram illustrating an example scanning process of an imaging method based on point scan;
FIG. 1B is a schematic diagram illustrating an example scanning process of an imaging method based on line scan;
FIG. 2 is a flowchart illustrating a super-resolution imaging method based on line scan according to some embodiments of the present disclosure;
FIG. 3A is a schematic diagram illustrating an example scanning process of a super-resolution imaging method based on line scan according to some embodiments of the present disclosure;
FIG. 3B is a schematic diagram illustrating illumination modes for rows that have been scanned in FIG. 3A in combination for explanatory purposes;
FIG. 4 is a schematic diagram illustrating illumination modes for rows that have been scanned in an example scanning process of a super-resolution imaging method based on line scan according to some other embodiments of the present disclosure in combination for explanatory purposes;
FIG. 5 is a schematic diagram illustrating illumination modes for rows that have been scanned in an example scanning process of a super-resolution imaging method based on line scan according to yet some other embodiments of the present disclosure in combination for explanatory purposes;
FIG. 6A is a schematic diagram illustrating an example scanning process of a super-resolution imaging method based on line scan according to some other embodiments of the present disclosure;
FIG. 6B is a schematic diagram illustrating illumination modes for rows that have been scanned in FIG. 6A in combination for explanatory purposes;
FIG. 7 is a schematic diagram illustrating an example scanning process of a super-resolution imaging method based on line scan according to yet some other embodiments of the present disclosure;
FIG. 8 is a schematic block diagram illustrating a super-resolution imaging apparatus based on line scan according to some embodiments of the present disclosure;
FIG. 9A is a schematic block diagram illustrating an illumination module of FIG. 8 according to some embodiments of the present disclosure;
FIG. 9B is a schematic block diagram illustrating an illumination module of FIG. 8 according to some other embodiments of the present disclosure;
FIG. 10 is an example optical path diagram illustrating an illumination module of FIG. 8 according to some embodiments of the present disclosure;
FIG. 11 is an example optical path diagram illustrating a detection module and a scanning module of FIG. 8 according to some embodiments of the present disclosure;
   a part A of FIG. 12 shows optical intensity distribution diagrams in three two-dimensional sections xy, zy, and xz that are formed respectively by structured line excitation light 1 and structured line excitation light 2 provided by the illumination module of FIG. 10 on a sample at a focal plane of a microscope objective of FIG. 11, and a part B of FIG. 12 shows optical intensity distribution curves along selected section lines y1, x2, y2, z1, and z2 in the optical intensity distribution diagrams shown in the part A of FIG. 12;

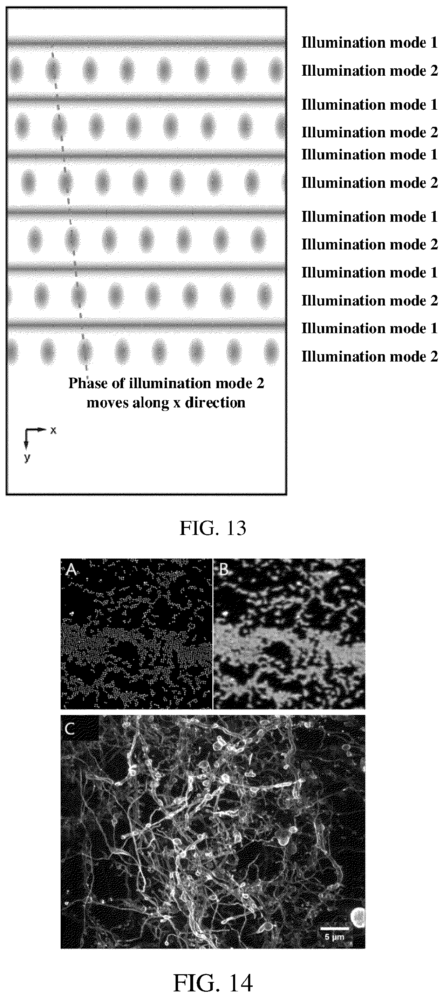

FIG. 13 is schematic diagram illustrating illumination modes for rows that have been scanned using the structured line excitation light 1 and structured line excitation light 2 provided by the illumination module of FIG. 10 in combination for explanatory purposes;
   a part A of FIG. 14 shows an imaging result of fluorescent microbeads using the super-resolution imaging technique of the present disclosure, a part B of FIG. 14 shows an imaging result of the fluorescent microbeads in the same area as that shown in the part A of FIG. 14 using a conventional confocal microscope, and a part C of FIG. 14 shows an imaging result of neurons in a brain of an awake mouse using the super-resolution imaging technique of the present disclosure.

It is to be noted that in the embodiments illustrated below, sometimes the same reference signs are commonly used across different accompanying drawings to represent the same parts or parts with the same function, and repeated descriptions thereof are omitted. In some cases, similar items are indicated using similar reference numbers and letters, and thus, once a certain item is defined in one drawing, it need not be discussed further in subsequent drawings.

For ease of understanding, positions, dimensions, ranges, or the like of structures shown in the accompanying drawings or the like sometimes do not represent practical positions, dimensions, ranges, or the like. Therefore, the present disclosure is not limited to the positions, dimensions, ranges, or the like disclosed in the accompanying drawings or the like.

### DETAILED DESCRIPTION

Various exemplary embodiments of the present disclosure will be described below in detail with reference to the accompanying drawings. It should be noted that: unless otherwise specifically illustrated, the relative arrangement of components and steps, numerical expressions, and numerical values set forth in these embodiments do not limit the scope of the present disclosure.

In fact, the following descriptions of at least one exemplary embodiment are merely illustrative, and in no way constitute any limitation on the present disclosure and the application or use thereof. In other words, the structure and the method herein are shown in an exemplary manner to illustrate different embodiments of the structure and the method in the present disclosure. However, a person skilled in the art will understand that they merely illustrate exemplary manners rather than exhaustive manners in which the present disclosure may be implemented. Moreover, the accompanying drawings are not necessarily drawn to scale, and some features may be enlarged to show details of specific components.

In addition, a technology, a method, and a device known to a person of ordinary skill in the related art may not be discussed in detail, but in proper circumstances, the technology, method, and device shall be regarded as a part of the specification.

In all examples that are shown and discussed herein, any specific value should be interpreted only as an example but not as a limitation. Therefore, there may be different values in other examples of the exemplary embodiments.

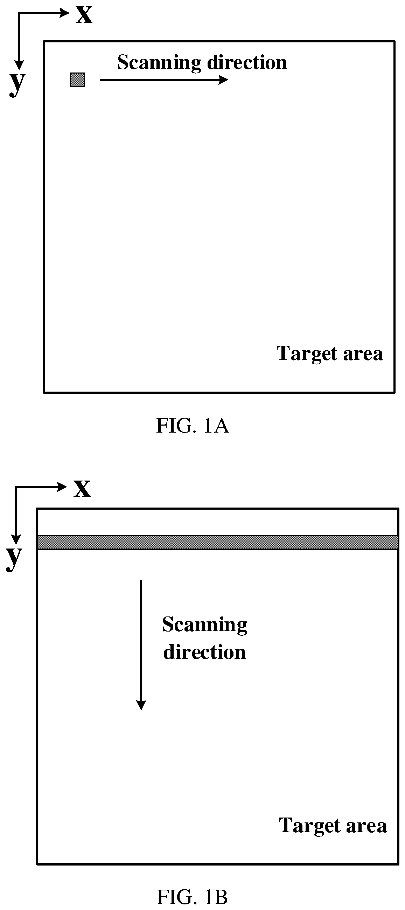

Among existing super-resolution imaging techniques, super-resolution imaging techniques based on point scan have been proposed, such as stimulated emission depletion (STED) super-resolution imaging technique, point-scan structured-light-illumination super-resolution imaging technique, etc. For example, referring to FIG. 1A, during the point scan, information is collected serially through point-by-point scanning of a target area, and then the information collected at each point in each row is reconstructed into an image of the target area. Therefore, the imaging speed of the super-resolution imaging technique based on point scan is usually low. If the target area is displaced when a certain row is scanned point by point compared to when an adjacent previous row is scanned point by point, then pixels of the two rows are misplaced, resulting in the inability to obtain the correct image information. Therefore, the super-resolution imaging technique based on point scan, because of its low imaging speed, often can only tolerate a very low-speed movement of the target area during the imaging or cannot tolerate any movement of the target area during the imaging at all.

Super-resolution imaging techniques based on wide-field imaging have been also proposed, such as structured-light-illumination super-resolution imaging technique, single-molecule positioning imaging technique, etc. The super-resolution imaging technique based on wide-field imaging requires multiple imaging of the entire target area to capture a plurality of full-field two-dimensional images, and finally the plurality of full-field two-dimensional images are synthesized into a super-resolution image through calculations. However, this technique requires the target area to stay stationary during the entire imaging process, otherwise it is impossible to reconstruct the super-resolution image.

The current mainstream super-resolution imaging techniques usually require the sample or more specifically the target area therein to stay stationary, and they are also very sensitive to even minor movements of the target area during the imaging process. In view of this, in biological researches where imaging technology is widely used, the existing super-resolution imaging techniques are mostly used to image cultured cells, but it is difficult to expand to image live animals. This is because the physiological activities such as breathing and heartbeat of live animals (for example, mice) will inevitably cause minor movements of the target area. In some experimental requirements, it is even required to image live animals in awake and active states. During this process, the limb movement of the live animals causes a significant movement of the target area, making the existing super-resolution imaging techniques inapplicable.

Therefore, an improved super-resolution imaging technique that can tolerate the movement of the target area during the imaging process is needed.

The inventors of the present disclosure have noticed that line scan has a faster speed than the point scan. For example, referring to FIG. 1B, during the line scan, an entire row (in an x direction) in the target area is illuminated by excitation light simultaneously, and a detection apparatus such as a camera is used to detect the entire row simultaneously. Then, by scanning row by row along a y direction, the entire target area can be imaged in two dimensions. Therefore, comparing the point scanning process shown in FIG. 1A with the line scanning process shown in FIG. 1B, it can be found that the imaging speed based on line scan is much faster than the imaging speed based on point scan. However, traditional confocal imaging techniques based on line scan can only obtain an image with a resolution of diffraction limit through a single scan. In order to achieve a resolution exceeding the limitation of diffraction limit, the existing super-resolution imaging techniques (for example, the aforementioned super-resolution imaging technique based on wide-field imaging) usually need to obtain multiple imaging results through multiple scans and then integrate them into a super-resolution image. However, the time interval between two adjacent scans is long, making the imaging speed of such super-resolution imaging technique also low.

To this end, the present disclosure proposes a super-resolution imaging technique based on line scan, which can obtain a super-resolution image through a single scan, without repeating multiple scans and then integrating multiple imaging results. The super-resolution imaging technique based on line scan of the present disclosure has a higher imaging speed, thereby having higher tolerance to the movement of the target area during the imaging process.

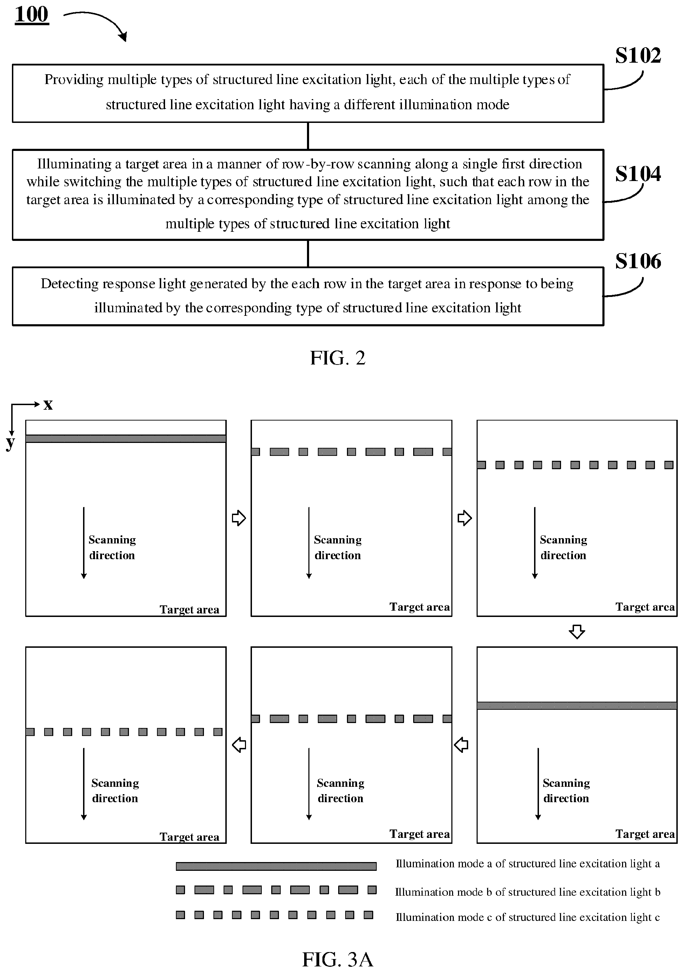

A super-resolution imaging method 100 based on line scan (hereinafter referred to as method 100) according to some embodiments of the present disclosure is first described below in detail with reference to FIG. 2. As shown in FIG. 2, the method 100 includes: at step S102, providing multiple types of structured line excitation light, each of the multiple types of structured line excitation light having a different illumination mode; at step S104, illuminating a target area in a manner of row-by-row scanning along a single first direction (for example, the y direction shown in FIG. 3A) while switching the multiple types of structured line excitation light, such that each row in the target area is illuminated by a corresponding type of structured line excitation light among the multiple types of structured line excitation light; and at step S106, detecting response light generated by the each row in the target area in response to being illuminated by the corresponding type of structured line excitation light.

Herein, the "structured line excitation light" refers to excitation light with linear structure, whose projection on the target area exhibits a linear distribution. Herein, the "illumination mode" of the "structured line excitation light" refers to a spatial distribution pattern of optical intensity of the structured line excitation light. Or more specifically, it can refer to the pattern of the projection of the structured line excitation light on the target area (this can reflect the optical intensity distribution of the structured line excitation light on a two-dimensional section in a plane where the target area is located). It can be understood that if the patterns of projections of two beams of light on the target area are completely coincident with each other or can be completely coincident with each other after translation, then the illumination modes of the two beams of light can be considered the same. Based on fluorescence imaging principle, after being illuminated by "excitation light", the target area will accordingly emit "response light", and the target area can be imaged according to the "response light". Herein, the "target area" is an area to be imaged.

According to the invention, the multiple types of structured line excitation light provided at step S102 include first structured line excitation light and second structured line excitation light. The first structured line excitation light may have a illumination mode that completely illuminates an entire row, and the second structured line excitation light may have an illumination mode that does not completely illuminate an entire row. For example, the first structured line excitation light may have a spot extending continuously in a second direction (e.g., the x direction shown in FIG. 3A) perpendicular to the first direction (e.g., the y direction shown in FIG. 3A) (e.g., structured line excitation light a with an illumination mode a shown in FIG. 3A). The second structured line excitation light may have a plurality of spots arranged at substantially periodic intervals in the second direction (e.g., the x direction shown in FIG. 3A) (e.g., structured line excitation light b with an illumination mode b and structured line excitation light c with an illumination mode c shown in FIG. 3A). Herein, being arranged at "substantially" periodic intervals refers to a change in the intervals is within a range of ±20% of a design interval period or a target interval period, or for example within a range of ±15%, or for example within a range of ±10%, or for example within a range of ±5%, etc. In some embodiments, the multiple types of structured line excitation light provided at step S102 may further additionally or alternatively include multiple types of such second structured line excitation light, wherein each type of second structured line excitation light compared to other second structured line excitation light may have a different combination of a spot shape and an interval period. For example, the spot shape and the interval period of the structured line excitation light b with the illumination mode b shown in FIG. 3A are different from the spot shape and the interval period of the structured line excitation light c with the illumination mode c shown in FIG. 3A. Herein, the difference in the interval periods can be understood as the interval periods are not the same or not "substantially" the same. For example, the interval periods may differ by more than ±20%, or for example more than ±50%, or for example more than ±70%, etc. In some other embodiments, the second structured line excitation light may also have a plurality of spots arranged at aperiodic intervals in the second direction (e.g., the x direction shown in FIG. 3A).

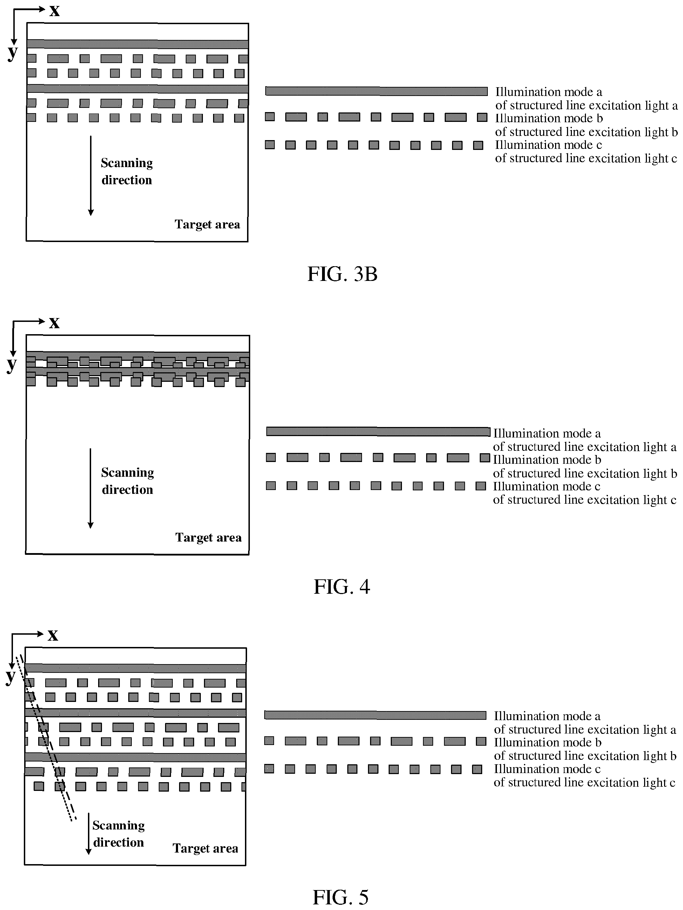

As a non-limiting example, FIG. 3A and FIG. 3B show three types of structured line excitation light with different illumination modes, that is, the structured line excitation light a with the illumination mode a, the structured line excitation light b with the illumination mode b, and the structured line excitation light c with the illumination mode c. It can be understood that, any suitable number of types of structured line excitation light can be set as needed, including but not limited to two, three, four or more.

FIG. 3A illustratively shows how the steps S104 and S106 are implemented: (1) first, the scan starts with a first row of the target area, the first row is illuminated by using the structured line excitation light a with the illumination mode a, and response light generated by the first row in response to the first row being illuminated by the structured line excitation light a is detected; (2) then the scan proceeds along the y direction to a second row of the target area, the second row is illuminated by switching to use the structured line excitation light b with the illumination mode b, and response light generated by the second row in response to the second row being illuminated by the structured line excitation light b is detected; (3) then the scan proceeds along the y direction to a third row of the target area, the third row is illuminated by switching to use the structured line excitation light c with the illumination mode c, and response light generated by the third row in response to the third row being illuminated by the structured line excitation light c is detected; (4) then the scan proceeds along the y direction to a fourth row of the target area, the fourth row is illuminated by switching to use the structured line excitation light a with the illumination mode a, and response light generated by the fourth row in response to the fourth row being illuminated by the structured line excitation light a is detected; (5) then the scan proceeds along the y direction to a fifth row of the target area, the fifth row is illuminated by switching to use the structured line excitation light b with the illumination mode b, and response light generated by the fifth row in response to the fifth row being illuminated by the structured line excitation light b is detected; (6) then the scan proceeds along the y direction to a sixth row of the target area, the sixth row is illuminated by switching to use the structured line excitation light c with the illumination mode c, and response light generated by the sixth row in response the sixth row to being illuminated by the structured line excitation light c is detected; ... and so on, each subsequent row of the target area is successively illuminated by taking turns to use the structured line excitation light a, the structured line excitation light b and the structured line excitation light c and detected. Finally, after scanning the entire target area row by row, a super-resolution image can be reconstructed directly based on the detection results of the response light generated by the respective rows of the target area.

The multiple types of structured line excitation light provided at step S102 may be switched according to a preset order to illuminate corresponding rows in the target area. For example, in the embodiment shown in FIG. 3A, the multiple types of structured line excitation light provided at step S102 are traversed and used according to the same order in each round to illuminate the corresponding rows in the target area. Alternatively, the multiple types of structured line excitation light provided at step S102 may also be traversed and used according to a different order in each round to illuminate the corresponding rows in the target area. In some embodiments, it may only require that two adjacent rows in the target area are illuminated by different types of structured line excitation light among the multiple types of structured line excitation light. In some other embodiments, it is also possible to switch to a different type of structured line excitation light every few rows for illumination. In short, the switching order can be specifically designed according to the actual application scenario. The various switching orders such as "a, b, c, a, b, c......", "a, b, c, c, b, a, b, a, c......", "a, a, b, b, c, c, a, a, b, b, c, c...... ", "a, b, a, c, b, c......" or the like can all have their specific applicable application scenarios.

A time difference between scanning two rows may be limited by limitations of factors such as the detection speed of the detection apparatus (e.g., a camera) for the response light, the switching speed of the structured line excitation light with different illumination modes (if it is necessary to switch the structured line excitation light with different illumination modes), the scanning speed, etc. When permitted, the time difference can be set as short as possible, thereby improving the imaging speed of method 100 as much as possible.

For illustrative purposes, FIG. 3B virtually shows a combined representation of the six figures in FIG. 3A. In the embodiment of FIG. 3B, during the row-by-row scanning, regions illuminated by two consecutive applications of structured line excitation light in the target area do not overlap with each other. This is only an example rather than limiting. In some other embodiments, for example, as shown in FIG. 4, during the row-by-row scanning, the regions illuminated by two consecutive applications of structured line excitation light in the target area may partially overlap with each other. In some embodiments, a spacing between the regions illuminated by two consecutive applications of structured line excitation light in the target area in the first direction (e.g., the y direction) may be between 5% and 50% of the design resolution (e.g., 100 nm). In some embodiments, the row-by-row scanning can be performed at equal spacings. In some other embodiments, the row-by-row scanning can also be performed at unequal spacings.

In addition, in the embodiment of FIG. 3B, during the row-by-row scanning, the structured line excitation light that does not completely illuminate an entire row (for example, the aforementioned second structured line excitation light, such as the structured line excitation light b with the illumination mode b and the structured line excitation light c with the illumination mode c) is not subjected to any phase shift in the second direction (e.g., the x direction) each time it is used for illumination compared to last time it is used for illumination. In some other embodiments, for example, as shown in FIG. 5, during the row-by-row scanning, the structured line excitation light that does not completely illuminate an entire row (for example, the aforementioned second structured line excitation light, such as the structured line excitation light b with the illumination mode b and the structured line excitation light c with the illumination mode c) is subjected to a preset phase shift in the second direction (e.g., the x direction) each time it is used for illumination compared to last time it is used for illumination. When this structured line excitation light is the aforementioned second structured line excitation light, a phase-shift distance for the preset phase shift may be a non-integer multiple of the interval period of the spots of this structured line excitation light. Such phase shift may facilitate more fully collecting information from the target area for imaging.

In some embodiments, the multiple types of structured line excitation light provided at step S102 include third structured line excitation light and fourth structured line excitation light. An illumination mode of the third structured line excitation light can be configured to improve imaging performance in a third direction. An illumination mode of the fourth structured line excitation light can be configured to improve imaging performance in a fourth direction different from the third direction. In some examples, the third direction may be parallel to the first direction, and the fourth direction may be perpendicular to the third direction. In some examples, the illumination mode of the fourth structured line excitation light can be configured to improve imaging performance in both the fourth direction and a fifth direction that is perpendicular to both the third direction and the fourth direction. In some examples, the multiple types of structured line excitation light provided at step S102 may also include fifth structured line excitation light. An illumination mode of the fifth structured line excitation light can be configured to improve imaging performance in the fifth direction that is perpendicular to both the third direction and the fourth direction. For example, as shown in FIG. 12 described later, an illumination mode 1 of structured line excitation light 1 is configured to improve the imaging resolution in the y direction, and an illumination mode 2 of structured line excitation light 2 is configured to improve the imaging resolution in the x and z directions, such that the resulted image of the target area has high imaging resolution in each of the three directions, i.e., the x direction, the y direction, and the z direction.

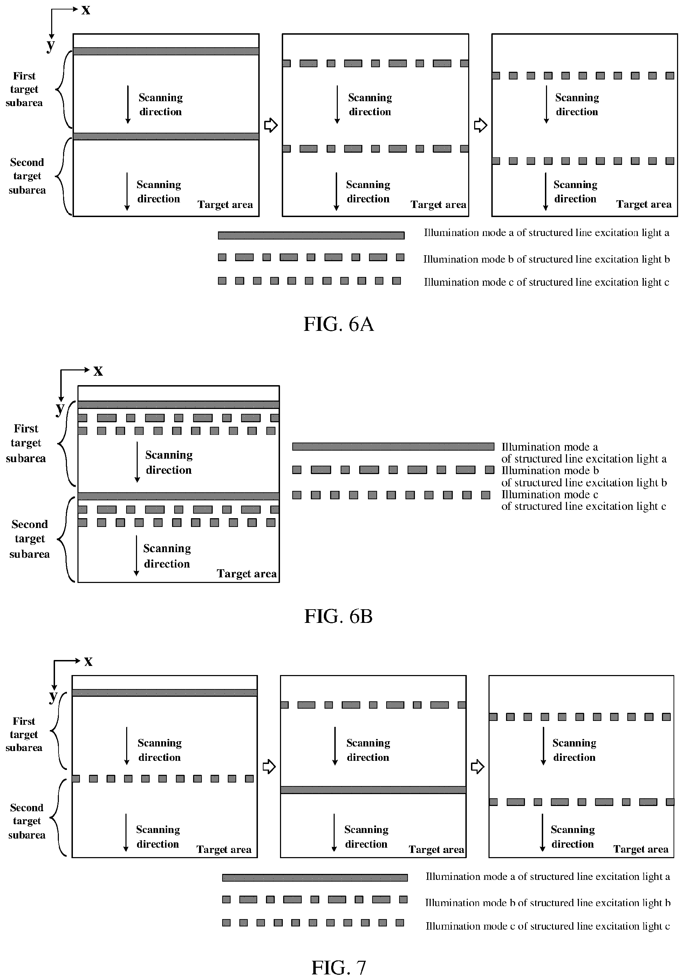

The imaging speed of the method 100 can also be further improved by a manner of parallel processing. For example, referring to FIG. 6A and FIG. 6B, in some embodiments, the target area may include multiple target subareas (e.g., a first target subarea and a second target subarea) arranged along the first direction (e.g., the y direction), and the method 100 includes performing the following operations on each target subarea in parallel: illuminating the target subarea in the manner of row-by-row scanning along the single first direction (e.g., the y direction) while switching the multiple types of structured line excitation light provided at step S102, such that each row in the target subarea is illuminated by a corresponding type of structured line excitation light among the multiple types of structured line excitation light; and detecting response light generated by the each row in the target subarea in response to being illuminated by the corresponding type of structured line excitation light. In the embodiment as shown in FIG. 6A, the scanning direction in the first target subarea is the same as the scanning direction in the second target subarea. In some other embodiments, the scanning direction in the first target subarea is different from the scanning direction in the second target subarea. For example, the scanning direction in the first target subarea may be opposite to the scanning direction in the second target subarea.

In some embodiments, a first row in the first target subarea and a second row in the second target subarea can be illuminated simultaneously. In some examples, for example, as shown in FIG. 6A, the first row and the second row can be illuminated by the same type of structured line excitation light among the multiple types of structured line excitation light. In some other examples, for example, as described in FIG. 7, the first row and the second row can be illuminated by different types of structured line excitation light among the multiple types of structured line excitation light. It can be understood that, although the examples in FIG. 6A to FIG. 7 only illustrate that the target area is divided into two target subareas, the target area may be divided into any suitable number of target subareas with any suitable shape according to specific needs.

The method 100 requires only single line-scan imaging to correctly reconstruct a super-resolution image. It benefits from a high line-scan imaging speed, which can effectively improve the tolerance of the super-resolution imaging method to the movement of the target area during the imaging process. For example, for existing scientific grade cameras, if only 4 rows of pixels are used to image the target area, the frame rate can exceed 40 KHz. If it is desired that the displacement amount of the target area occurred during information collection from 10 consecutive rows is less than 50 nm (half the design resolution of 100 nm), then the movement speed of the target area that can be tolerated by the method 100 is as high as 200 micrometers per second. This is sufficient to meet the needs of most application scenarios (for example, a movement speed of a brain area of an anesthetized mouse is around 2 micrometers per second, and a movement speed of a brain area of an awake mouse is around 50 micrometers per second). Even if the movement of the target area is so violent that the obtained image may have been deformed on a large scale, it will not affect the ability of the method 100 to analyze fine structures on a micro scale.

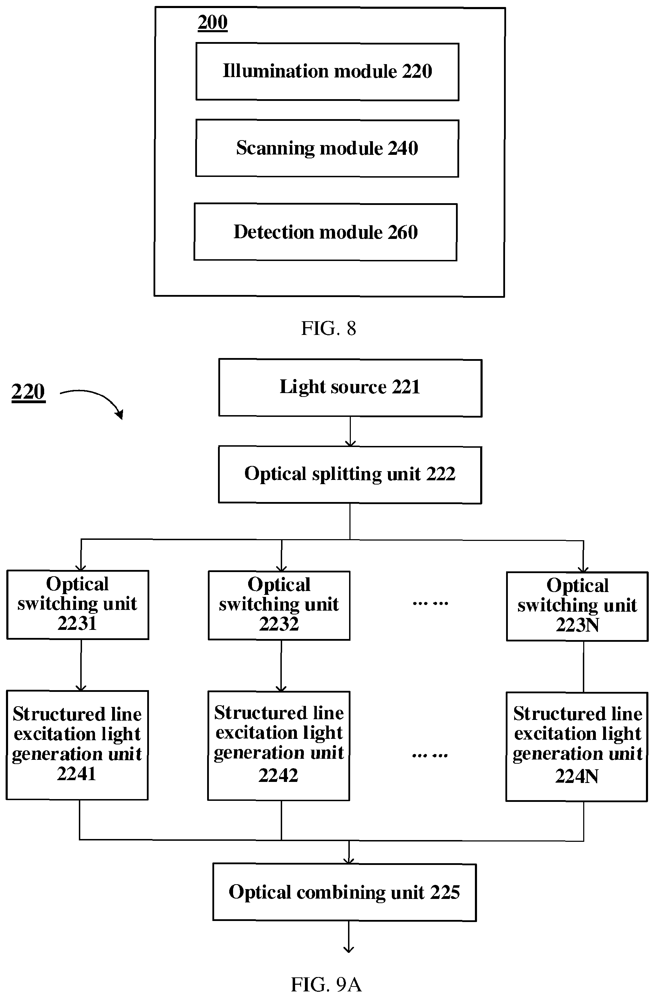

A super-resolution imaging apparatus 200 based on line scan (hereinafter referred to as apparatus 200) according to some embodiments of the present disclosure is described below in detail with reference to FIG. 8. As shown in FIG. 8, the apparatus 200 includes an illumination module 220, a scanning module 240 and a detection module 260. The illumination module 220 is configured to provide multiple types of structured line excitation light, each of the multiple types of structured line excitation light having a different illumination mode. The scanning module 240 is configured to illuminate a target area in a manner of row-by-row scanning along a single first direction while switching the multiple types of structured line excitation light, such that each row in the target area is illuminated by a corresponding type of structured line excitation light among the multiple types of structured line excitation light. The detection module 260 is configured to detect response light generated by the each row in the target area in response to being illuminated by the corresponding type of structured line excitation light.

In some embodiments, for example, referring to FIG. 9A, the illumination module 220 may include: a light source 221 configured to provide excitation light; an optical splitting unit 222 configured to split the excitation light from the light source 221 into multiple beams of excitation light; a plurality of structured line excitation light generation units 2241, 2242, ..., 224N, each structured line excitation light generation unit being configured to receive a corresponding beam of excitation light from the optical splitting unit 222 and generate a corresponding type of structured line excitation light based on the received corresponding beam of excitation light; an optical combining unit 225 configured to combine multiple types of structured line excitation light from the plurality of structured line excitation light generation units 2241, 2242, ... , 224N into a beam; and a plurality of optical switching units 2231, 2232, ... , 223N. Each optical switching unit, for example, may be disposed between the optical splitting unit 222 and a corresponding one of the structured line excitation light generation units 2241, 2242, ... , 224N, and be configured to control whether the excitation light from the optical splitting unit 222 is output to the corresponding one of the structured line excitation light generation units (for example, the case as shown in FIG. 9A). Or each optical switching unit may alternatively be disposed between a corresponding one of the structured line excitation light generation units 2241, 2242, ... , 224N and the optical combining unit 225, and be configured to control whether the structured line excitation light from the corresponding one of the structured line excitation light generation units is output to the optical combining unit 225. The structured line excitation light generated by different structured line excitation light generation units among the plurality of structured line excitation light generation units 2241, 2242, ... , 224N has different illumination modes.

In some other embodiments, for example, referring to FIG. 9B, the illumination module 220 may also include: a light source 221 configured to provide excitation light; a single modulation unit 226 configured to modulate the excitation light from the light source 221 into structured line excitation light with different illumination modes. The modulation unit 226 may include, for example, at least one of a spatial light modulator or a polarization modulator, etc., and may output structured line excitation light with different illumination modes according to setting.

Incidentally, the excitation light provided by the light source 221 may include one or more wavelengths or ranges of wavelengths, as long as it can excite the target area to generate response light, without being particularly limited.

The illumination module 220 as shown in FIG. 9B has a simpler structure and a smaller number of components than the illumination module 220 as shown in FIG. 9A. However, the illumination module 220 as shown in FIG. 9A can switch structured line excitation light with different illumination modes faster than the illumination module 220 as shown in FIG. 9B. It can be understood that, FIG. 9A and FIG. 9B are only illustrative rather than limiting, and any suitable illumination module 220 can be adopted or designed according to the teachings of the present disclosure to provide multiple types of structured line excitation light with different illumination modes.

According to the invention, , the multiple types of structured line excitation light provided by the illumination module 220 include first structured line excitation light and second structured line excitation light. The first structured line excitation light has a spot extending continuously in a second direction perpendicular to the first direction. The second structured line excitation light has a plurality of spots arranged at substantially periodic intervals in the second direction. In some embodiments, the multiple types of structured line excitation light may further additionally or alternatively include multiple types of second structured line excitation light having a plurality of spots arranged at substantially periodic intervals in the second direction perpendicular to the first direction. Each of the multiple types of second structured line excitation light has a different combination of a spot shape and an interval period compared to others of the multiple types of second structured line excitation light.

In some embodiments, the multiple types of structured line excitation light provided by the illumination module 220 include third structured line excitation light and fourth structured line excitation light. An illumination mode of the third structured line excitation light is configured to improve imaging performance in a third direction. An illumination mode of the fourth structured line excitation light is configured to improve imaging performance in a fourth direction different from the third direction. In some examples, the third direction is parallel to the first direction, and the fourth direction is perpendicular to the third direction. In some examples, the illumination mode of the fourth structured line excitation light is configured to improve imaging performance in both the fourth direction and a fifth direction that is perpendicular to both the third direction and fourth direction. In some embodiments, the multiple types of structured line excitation light further includes a fifth structured line excitation light. An illumination mode of the fifth structured line excitation light is configured to improve the imaging performance in the fifth direction that is perpendicular to both the third direction and the fourth direction.

In some embodiments, the scanning module 240 may further be configured such that, during the row-by-row scanning, the second structured line excitation light is subjected to a preset phase shift in the second direction each time it is used for illumination compared to last time it is used for illumination, with a phase-shift distance being a non-integer multiple of the interval period of the spots of the second structured line excitation light. In some embodiments, the scanning module 240 may be further configured to illuminate two adjacent rows in the target area with different types of structured line excitation light among the multiple types of structured line excitation light. In some embodiments, the scanning module 240 may be further configured to switch the multiple types of structured line excitation light according to a preset order to illuminate respective rows in the target area. In some embodiments, the scanning module 240 may be further configured such that, during the row-by-row scanning, regions illuminated by two consecutive applications of structured line excitation light in the target area partially overlap with each other.

In order to further improve the imaging speed of the apparatus 200, the target area can be divided into multiple target subareas and then parallel processing can be performed. In some embodiments, the target area may include multiple target subareas arranged along the first direction. The scanning module 240 may be configured to perform the following operations on each target subarea in parallel: illuminating a target subarea in the manner of row-by-row scanning along the single first direction while switching the multiple types of structured line excitation light, such that each row in the target subarea is illuminated by a corresponding type of structured line excitation light among the multiple types of structured line excitation light. The detection module 260 may be configured to perform the following operations on the each target subarea in parallel: detecting response light generated by the each row in the target subarea in response to being illuminated by the corresponding type of structured line excitation light. In some embodiments, the multiple target subareas may include a first target subarea and a second target subarea. A first row in the first target subarea and a second row in the second target subarea can be illuminated simultaneously. The scanning module 240 may be configured to illuminate the first row and the second row with the same or different types of structured line excitation light among the multiple types of structured line excitation light provided by the illumination module 220.

The embodiments of the apparatus 200 may be similar to the embodiments of the aforementioned method 100, which are not repeated herein.

For illustrative purposes, a non-limiting specific example in which the super-resolution imaging technique of the present disclosure is applied is described in detail below with reference to FIG. 10 to FIG. 14. It should be understood that there may be other optical elements in an actual optical system, and these other optical elements are neither discussed herein nor shown in the accompanying drawings in order not to obscure the key points herein.

An optical system as shown in FIG. 10 can be built to realize an illumination module that provides two types of structured line excitation light with different illumination modes. An input of the illumination module can be a parallel laser beam of a single wavelength or multiple wavelengths. The input light beam is split by a polarization beam splitter PBS1 (which can serve as the optical splitting unit) into two light beams with polarizations perpendicular to each other. These two light beams with polarizations perpendicular to each other are respectively controlled by two acousto-optic tunable filters AOTF1 and AOTF2 (which can serve as the optical switching units). The acousto-optic tunable filters AOTF1 and AOTF2 can selectively allow light of a specific wavelength or within a specific range of wavelengths to pass therethrough, and can quickly control the on-off of light. In the optical path on the left of FIG. 10, the parallel light beam transmitted from the polarization beam splitter PBS1 passes through the acousto-optic tunable filter AOTF1 and then is converged by a cylindrical lens CL1 into a line along the x direction, that is, the illumination mode 1 as shown in part A of FIG. 12. In the optical path on the right of FIG. 10, the parallel light beam reflected from the polarization beam splitter PBS1 is first reflected by a mirror M1 and then, after passing through the acousto-optic tunable filter AOTF2, is converged by a cylindrical lens CL2 into a line along the x direction. Different from the optical path on the left, a transmission grating TG driven by a rotating motor is disposed at a focal plane of the cylindrical lens CL2. The transmission grating TG modulates a phase or intensity of the linearly converged light emitted from the cylindrical lens CL2 along the x-direction, such that the light is modulated to have the illumination mode 2 as shown in part A of FIG. 12. In addition to using the transmission grating TG driven by the rotating motor, it is also possible to alternatively use other solutions such as a digital micromirror device (DMD), a liquid crystal spatial light modulator (SLM), etc. to modulate the phase or intensity of the light. The transmission grating TG driven by the rotating motor can change the phase faster than DMD and SLM, while DMD and SLM can simply enable phase changes without being driven by a rotating motor. Thus, the cylindrical lens CL1 can serve as a first structured line excitation light generation unit providing the structured line excitation light 1 with the illumination mode 1. The cylindrical lens CL2 and the transmission grating TG can serve as a second structured line excitation light generation unit providing the structured line excitation light 2 with the illumination mode 2. The structured line excitation light 1 with the illumination mode 1 and the structured line excitation light 2 with the illumination mode 2 (reflected by a mirror M2) are combined into a beam by a polarization beam splitter PBS2 (which can serve as the optical combining unit). In addition, a spherical lens L1 and a spherical lens L3 constitute a relay imaging system to image the structured line excitation light 1 with the illumination mode 1 at a focal plane of the spherical lens L3. A spherical lens L2 and the spherical lens L3 also constitute a relay imaging system to image the structured line excitation light 2 with the illumination mode 2 at the focal plane of the spherical lens L3. Thus, by controlling the acousto-optic tunable filters AOTF1 and AOTF2, the illumination module as shown in FIG. 10 can selectively output the structured line excitation light 1 with the illumination mode 1 or the structured line excitation light 2 with the illumination mode 2.

An optical system as shown in FIG. 11 can be built to realize the scanning module and the detection module, in which solid-line arrows schematically indicate the optical path of the scanning module, and dotted-line arrows schematically indicate the optical path of the detection module. A spherical lens L4 and a spherical lens L5 may constitute a relay imaging system. A spherical lens L6 and a microscopic objective may also constitute a relay imaging system. The structured line excitation light output by the illumination module is reflected by a dichroic mirror DM, then passes through the spherical lens L4 to reach a scanning mirror SM. After being reflected by the scanning mirror SM, it passes through the spherical lens L5, the spherical lens L6, and the microscopic objective to reach a target area of a sample. In the implementation shown in FIG. 11, the scanning of the illumination position of the structured line excitation light in the target area is achieved through the rotation of the scanning mirror SM. In this case, the sample can be fixed. Additionally or alternatively, the sample can be disposed on a displacement stage for sample scanning, and the scanning of the illumination position of the structured line excitation light in the target area is achieved through the displacement of the sample. In this case, the scanning mirror SM can still be used, or it can be replaced with a fixed mirror. Response light emitted by the sample is collected by the microscopic objective, then passes through the spherical lens L6, the spherical lens L5, the scanning mirror SM, the spherical lens L4, and the dichroic mirror DM, and finally is captured by a camera. In addition to using the camera, it is also possible to alternatively use other solutions such as a photoelectric detector, an image sensor, etc. to detect the response light. In the embodiment as shown in FIG. 11, the scanning module and the detection module share most of the optical elements, which can be integrated in a single module called line-scan confocal imaging module.

Referring to FIG. 12, it specifically shows the illumination modes of the structured line excitation light 1 and the structured line excitation light 2 provided by the illumination module of FIG. 10 and projected by the scanning module of FIG. 11 on the target area of the sample. As shown in FIG. 12, the illumination mode 1 is a line that is uniform in the x direction and converged in the y direction to the greatest extent, which produces the fastest change in the intensity in the y direction, thereby improving the imaging resolution in the y direction. The illumination mode 2 is a row of spots arranged at substantially periodic intervals along the x direction, which produces the fastest changes in the intensities in the x and z directions, thereby improving the imaging resolution in the x and z directions.

During the line scanning process, by controlling the alternating on-and-off of the acousto-optic tunable filters AOTF1 and AOTF2 in the illumination module of FIG. 10, alternating illumination of the target area of the sample by the structured line excitation light 1 with the illumination mode 1 and the structured line excitation light 2 with the illumination mode 2 is achieved. During this period, the rotating motor can also be used to rotate the transmission grating TG in the illumination module of FIG. 10, so that the phase of the illumination mode 2 of the structured line excitation light 2 continuously moves along the x direction, for example, referring to FIG. 13. The information collected for the target area in this manner includes high-resolution information in the three directions of x, y, and z, so that the image of the target area with high resolution in each of the three directions of x, y, and z can be reconstructed with super-resolution.

Referring to FIG. 14, part A of FIG. 14 shows an imaging result of fluorescent microbeads using the super-resolution imaging technique of the present disclosure, part B of FIG. 14 shows an imaging result of the fluorescent microbeads in the same area shown in part A using a conventional confocal microscope, and part C of FIG. 14 shows an imaging result of neurons in a brain of an awake mouse using the super-resolution imaging technique of the present disclosure. It can be seen that the imaging performance of the super-resolution imaging technique of the present disclosure is significantly better than that of the traditional imaging technique, and can resist the movement of the target area during the imaging process, which is very suitable for imaging live animals.

The terms "left", "right", "front", "rear", "top", "bottom", "above", "under", "upper", "lower", and the like in the specification and the claims, if present, are used for a descriptive purpose and are not necessarily used for describing an unchanged relative position. It is to be understood that the words used in such a way are interchangeable in proper circumstances so that the embodiments of the present disclosure described herein, for example, can be operated in other orientations that are different from those shown herein or those described otherwise. For example, when the apparatus in the accompanying drawings is turned upside down, a feature originally described as being "above" another feature may be described as being "under" another feature in this case. The apparatus may also be otherwise oriented (rotated 90 degrees or at other orientations), and in this case, a relative spatial relationship will be interpreted accordingly.

In the specification and the claims, when an element is referred to as being "above" another element, "attached" to another element, "connected" to another element, "coupled" to another element, "linked" to another element, "in contact" with another element, or the like, the element may be directly above the another element, directly attached to the another element, directly connected to the another element, directly coupled to the another element, directly linked to the another element, or in direct contact with the another element; or one or more intermediate elements may exist. In contrast, when an element is referred to as being "directly above" another element, "directly attached" to another element, "directly connected" to another element, "directly coupled" to another element, "directly linked" to another element, or "in direct contact" with another element, no intermediate element exists. In the specification and the claims, a feature being arranged as being "adjacent" to another feature may mean that the feature has a part that overlaps with the adjacent feature or that is located above or under the adjacent feature.

As used herein, the term "exemplary" means "used as an example, instance, or illustration", and not as a "model" to be accurately copied. Any implementation exemplarily described herein is not necessarily to be construed as preferred or advantageous over other implementations. In addition, the present disclosure is not limited by any stated or implied theory provided in the technical field, background, summary, or detailed description.

As used herein, the term "substantially" means that any minor variation caused by a defect of a design or manufacturing, a tolerance of a device or an element, environmental impact, and/or other factors is included. The term "substantially" also allows for a difference from a perfect or ideal situation caused by parasitic effect, noise, and other practical consideration factors that may exist in practical implementation.

In addition, terms like "first" and "second" may also be used herein for a reference purpose only, and therefore are not intended for a limitation. For example, the terms "first", "second" and other such numerical terms relating to a structure or an element do not imply a sequence or an order unless the context clearly indicates otherwise.

It is to be further understood that the term "comprise/include", when used herein, specifies the presence of stated features, integers, steps, operations, units, and/or components, but do not preclude the presence or addition of one or more other features, integers, steps, operations, units, and/or components, and/or combinations thereof.

In the present disclosure, the term "provide" is used broadly for covering all manners of obtaining an object. Therefore, "providing an object" includes but is not limited to "purchasing", "preparing/manufacturing", "arranging/setting", "installing/assembling", and/or "ordering" the object, etc.

As used herein, the term "and/or" includes any and every combination of one or more of associated listed items. The terms used herein are merely for the purpose of describing specific embodiments but not intended to limit the present disclosure. The singular forms "a", "an", and "the" as used herein are intended to include plural forms as well, unless otherwise clearly stated in the context.

A person skilled in the art should appreciate that the boundaries between the operations as described above are merely illustrative. A plurality of operations may be combined into a single operation, a single operation may be distributed in an additional operation(s), and operations may be performed at least partially overlapping in time. In addition, alternative embodiments may include a plurality of instances of a specific operation, and an operation order may be changed in various other embodiments. Other modifications, changes, and replacements, however, are also possible. Therefore, the specification and the accompanying drawings are to be regarded as illustrative rather than restrictive.

Although some specific embodiments of the present disclosure are described in detail by examples, the scope of the present disclosure is defined by the appended claims.

## Claims

1. A super-resolution imaging method (100) based on line scan, comprising:
providing (S102) structured line excitation light;
illuminating (S104), by the structured line excitation light, a target area in a manner of row-by-row scanning along a single first direction (y); and
detecting (S106) response light generated by each row in the target area in response to being illuminated by the structured line excitation light,
**characterized in that**:
multiple types of structured line excitation light are provided, and each of the multiple types of structured line excitation light has a different spatial distribution pattern of optical intensity,
the method (100) further comprises switching the multiple types of structured line excitation light during the row-by-row scanning such that each row in the target area is illuminated by a corresponding type of structured line excitation light among the multiple types of structured line excitation light, and
the detected response light is generated by the each row in the target area in response to being illuminated by the corresponding type of structured line excitation light.

2. The super-resolution imaging method (100) of claim 1, wherein the multiple types of structured line excitation light comprise first structured line excitation light and second structured line excitation light, the first structured line excitation light has a spot extending continuously in a second direction (x) perpendicular to the first direction (y), and the second structured line excitation light has a plurality of spots arranged at substantially periodic intervals in the second direction (x),
wherein the substantially periodic intervals are intervals with a change from a design interval period or a target interval period being in a range of ±20% of the design interval period or the target interval period.

3. The super-resolution imaging method (100) of claim 1 or 2, wherein the multiple types of structured line excitation light comprise multiple types of second structured line excitation light that has a plurality of spots arranged at substantially periodic intervals in a second direction (x) perpendicular to the first direction (y), and each of the multiple types of second structured line excitation light has a different combination of a spot shape and an interval period compared to others of the multiple types of second structured line excitation light,
wherein the substantially periodic intervals are intervals with a change from a design interval period or a target interval period being in a range of ±20% of the design interval period or the target interval period.

4. The super-resolution imaging method (100) of claim 2, wherein, during the row-by-row scanning, the second structured line excitation light is subjected to a preset phase shift in the second direction (x) each time it is used for illumination compared to last time it is used for illumination, with a phase-shift distance being a non-integer multiple of an interval period of the spots of the second structured line excitation light.

5. The super-resolution imaging method (100) of claim 1, wherein the multiple types of structured line excitation light comprise third structured line excitation light and fourth structured line excitation light, a spatial distribution pattern of optical intensity of the third structured line excitation light is configured to improve imaging performance in a third direction, and a spatial distribution pattern of optical intensity of the fourth structured line excitation light is configured to improve imaging performance in a fourth direction different from the third direction.

6. The super-resolution imaging method (100) of claim 5, wherein the third direction is parallel to the first direction (y), and the fourth direction is perpendicular to the third direction.

7. The super-resolution imaging method (100) of claim 6, wherein the spatial distribution pattern of optical intensity of the fourth structured line excitation light is configured to improve imaging performance in both the fourth direction and a fifth direction that is perpendicular to both the third direction and the fourth direction.

8. The super-resolution imaging method (100) of claim 6, wherein the multiple types of structured line excitation light further comprise fifth structured line excitation light, and a spatial distribution pattern of optical intensity of the fifth structured line excitation light is configured to improve imaging performance in a fifth direction that is perpendicular to both the third direction and the fourth direction.

9. The super-resolution imaging method (100) of claim 1, wherein the multiple types of structured line excitation light are switched according to a preset order to illuminate respective rows in the target area.

10. The super-resolution imaging method (100) of claim 1, wherein, during the row-by-row scanning, regions illuminated by two consecutive applications of structured line excitation light in the target area partially overlap with each other.

11. The super-resolution imaging method (100) of claim 1, wherein the target area comprises multiple target subareas arranged along the first direction (y), and wherein the method (100) comprises performing the following operations on each of the multiple target subareas in parallel:
illuminating the target subarea in the manner of row-by-row scanning along the single first direction (y) while switching the multiple types of structured line excitation light, such that each row in the target subarea is illuminated by a corresponding type of structured line excitation light among the multiple types of structured line excitation light; and
detecting response light generated by the each row in the target subarea in response to being illuminated by the corresponding type of structured line excitation light.

12. A super-resolution imaging apparatus (200) based on line scan, comprising:
an illumination module (220) configured to provide structured line excitation light;
a scanning module (240) configured to illuminate, by the structured line excitation light, a target area in a manner of row-by-row scanning along a single first direction (y); and
a detection module (260) configured to detect response light generated by each row in the target area in response to being illuminated by the structured line excitation light,
**characterized in that**:
the illumination module (220) is configured to provide multiple types of structured line excitation light, each of the multiple types of structured line excitation light having a different spatial distribution pattern of optical intensity,
the scanning module (240) is further configured to switch the multiple types of structured line excitation light during the row-by-row scanning such that each row in the target area is illuminated by a corresponding type of structured line excitation light among the multiple types of structured line excitation light, and
the detected response light is generated by the each row in the target area in response to being illuminated by the corresponding type of structured line excitation light.

13. The super-resolution imaging apparatus (200) of claim 12, wherein the multiple types of structured line excitation light comprise first structured line excitation light and second structured line excitation light, the first structured line excitation light has a spot extending continuously in a second direction (x) perpendicular to the first direction (y), and the second structured line excitation light has a plurality of spots arranged at substantially periodic intervals in the second direction (x),
wherein the substantially periodic intervals are intervals with a change from a design interval period or a target interval period being in a range of ±20% of the design interval period or the target interval period, and
wherein the scanning module (240) is further configured to, during the row-by-row scanning, cause the second structured line excitation light to be subjected to a preset phase shift in the second direction (x) each time it is used for illumination compared to last time it is used for illumination, with a phase-shift distance being a non-integer multiple of an interval period of the spots of the second structured line excitation light.

14. The super-resolution imaging apparatus (200) of claim 12, wherein the illumination module (220) comprises:
a light source (221) configured to provide excitation light;
an optical splitting unit (222) configured to split the excitation light from the light source (221) into multiple beams of excitation light;
a plurality of structured line excitation light generation units (2241, 2242, ..., 224N), each of the plurality of structured line excitation light generation units (2241, 2242, ..., 224N) being configured to receive a corresponding beam of excitation light from the optical splitting unit (222) and to generate a corresponding type of structured line excitation light based on the received corresponding beam of excitation light;
an optical combining unit (225) configured to combine multiple types of structured line excitation light from the plurality of structured line excitation light generation units (2241, 2242, ..., 224N) into a beam; and
a plurality of optical switching units (2231, 2232, ..., 223N), each of the plurality of optical switching units (2231, 2232, ..., 223N) being disposed between the optical splitting unit (222) and a corresponding one of the structured line excitation light generation units (2241, 2242, ..., 224N) and being configured to control whether the excitation light from the optical splitting unit (222) is output to the corresponding one of the structured line excitation light generation units (2241, 2242, ..., 224N), or each of the plurality of optical switching units (2231, 2232, ..., 223N) being disposed between a corresponding one of the structured line excitation light generation units (2241, 2242, ..., 224N) and the optical combining unit (225) and being configured to control whether the structured line excitation light from the corresponding one of the structured line excitation light generation units (2241, 2242, ..., 224N) is output to the optical combining unit (225),
wherein the structured line excitation light generated by different structured line excitation light generation units (2241, 2242, ..., 224N) among the plurality of structured line excitation light generation units (2241, 2242, ..., 224N) has different spatial distribution patterns of optical intensity.

15. The super-resolution imaging apparatus (200) of claim 12, wherein the illumination module comprises:
a light source (221) configured to provide excitation light; and
a single modulation unit (226) configured to modulate the excitation light from the light source (221) into structured line excitation light with different spatial distribution patterns of optical intensity.

## Patentansprüche

1. Hochauflösendes Bildgebungsverfahren (100) auf Basis von Zeilenabtastung, umfassend:
Bereitstellen (S102) von strukturiertem Zeilenanregungslicht;
Beleuchten (S104), mit dem strukturierten Zeilenanregungslicht, eines Zielbereichs auf eine Weise eines Reihe-für-Reihe-Abtastens entlang einer einzelnen ersten Richtung (y); und
Detektieren (S106) von Reaktionslicht, das von jeder Reihe im Zielbereich als Reaktion darauf erzeugt wird, dass sie mit dem strukturierten Zeilenanregungslicht beleuchtet wird,
**dadurch gekennzeichnet, dass**:
mehrere Typen von strukturiertem Zeilenanregungslicht bereitgestellt werden und jeder von den mehreren Typen von strukturiertem Zeilenanregungslicht ein unterschiedliches räumliches Verteilungsmuster der optischen Intensität aufweist,
das Verfahren (100) ferner das Schalten der mehreren Typen von strukturiertem Zeilenanregungslicht während des Reihe-für-Reihe-Abtastens derart umfasst, dass jede Reihe im Zielbereich mit einem entsprechenden Typ von strukturiertem Zeilenanregungslicht von den mehreren Typen von strukturiertem Zeilenanregungslicht beleuchtet wird, und
das detektierte Reaktionslicht von jeder Reihe im Zielbereich als Reaktion darauf erzeugt wird, dass sie mit dem entsprechenden Typ von strukturiertem Zeilenanregungslicht beleuchtet wird.

2. Hochauflösendes Bildgebungsverfahren (100) nach Anspruch 1, wobei die mehreren Typen von strukturiertem Zeilenanregungslicht ein erstes strukturiertes Zeilenanregungslicht und ein zweites strukturiertes Zeilenanregungslicht umfassen, wobei das erste strukturierte Zeilenanregungslicht einen Lichtfleck aufweist, der sich ununterbrochen in einer zweiten Richtung (x) senkrecht zur ersten Richtung (y) erstreckt, und das zweite strukturierte Zeilenanregungslicht eine Vielzahl von Lichtflecken aufweist, die in im Wesentlichen periodischen Intervallen in der zweiten Richtung (x) angeordnet sind,
wobei die im Wesentlichen periodischen Intervalle Intervalle mit einer Änderung von einer vorgegebenen Intervallperiode oder einer Zielintervallperiode sind, die in einem Bereich von ± 20 % der vorgegebenen Intervallperiode oder der Zielintervallperiode liegt.

3. Hochauflösendes Bildgebungsverfahren (100) nach Anspruch 1 oder 2, wobei die mehreren Typen von strukturiertem Zeilenanregungslicht mehrere Typen von zweitem strukturiertem Zeilenanregungslicht umfassen, das eine Vielzahl von Lichtflecken aufweist, die in im Wesentlichen periodischen Intervallen in einer zweiten Richtung (x) senkrecht zur ersten Richtung (y) angeordnet sind, und jeder der mehreren Typen von zweitem strukturiertem Zeilenanregungslicht im Vergleich zu anderen der mehreren Typen von zweitem strukturiertem Zeilenanregungslicht eine unterschiedliche Kombination aus einer Lichtfleckform und einer Intervallperiode aufweist,
wobei die im Wesentlichen periodischen Intervalle Intervalle mit einer Änderung von einer vorgegebenen Intervallperiode oder einer Zielintervallperiode sind, die in einem Bereich von ± 20 % der vorgegebenen Intervallperiode oder der Zielintervallperiode liegt.

4. Hochauflösendes Bildgebungsverfahren (100) nach Anspruch 2, wobei während des Reihe-für-Reihe-Abtastens das zweite strukturierte Zeilenanregungslicht jedes Mal, wenn es zur Beleuchtung verwendet wird, im Vergleich zu seiner letzten Verwendung zur Beleuchtung, einer voreingestellten Phasenverschiebung in der zweiten Richtung (x) mit einem Phasenverschiebungsabstand unterzogen wird, der ein nicht ganzzahliges Vielfaches einer Intervallperiode der Lichtflecken des zweiten strukturierten Zeilenanregungslichts ist.

5. Hochauflösendes Bildgebungsverfahren (100) nach Anspruch 1, wobei die mehreren Typen von strukturiertem Zeilenanregungslicht drittes strukturiertes Zeilenanregungslicht und viertes strukturiertes Zeilenanregungslicht umfassen, ein räumliches Verteilungsmuster der optischen Intensität des dritten strukturierten Zeilenanregungslichts so ausgestaltet ist, dass es eine Bildgebungsleistung in einer dritten Richtung verbessert, und ein räumliches Verteilungsmuster der optischen Intensität des vierten strukturierten Zeilenanregungslichts so ausgestaltet ist, dass es eine Bildgebungsleistung in einer vierten Richtung verbessert, die sich von der dritten Richtung unterscheidet.

6. Hochauflösendes Bildgebungsverfahren (100) nach Anspruch 5, wobei die dritte Richtung parallel zur ersten Richtung (y) ist und die vierte Richtung senkrecht zur dritten Richtung ist.

7. Hochauflösendes Bildgebungsverfahren (100) nach Anspruch 6, wobei das räumliche Verteilungsmuster der optischen Intensität des vierten strukturierten Zeilenanregungslichts so ausgestaltet ist, dass es eine Bildgebungsleistung in sowohl der vierten Richtung als auch einer fünften Richtung verbessert, die senkrecht zu sowohl der dritten Richtung als auch der vierten Richtung ist.

8. Hochauflösendes Bildgebungsverfahren (100) nach Anspruch 6, wobei die mehreren Typen von strukturiertem Zeilenanregungslicht ferner fünftes strukturiertes Zeilenanregungslicht umfassen und ein räumliches Verteilungsmuster einer optischen Intensität des fünften strukturierten Zeilenanregungslichts so ausgestaltet ist, dass es eine Bildgebungsleistung in einer fünften Richtung verbessert, die senkrecht zu sowohl der dritten Richtung als auch der vierten Richtung ist.

9. Hochauflösendes Bildgebungsverfahren (100) nach Anspruch 1, wobei die mehreren Typen von strukturiertem Zeilenanregungslicht so gemäß einer voreingestellten Reihenfolge geschaltet werden, dass entsprechende Reihen im Zielbereich beleuchtet werden.

10. Hochauflösendes Bildgebungsverfahren (100) nach Anspruch 1, wobei während des Reihe-für-Reihe-Abtastens Regionen, die von zwei aufeinanderfolgenden Anwendungen von strukturiertem Zeilenanregungslicht im Zielbereich beleuchtet werden, einander teilweise überlappen.

11. Hochauflösendes Bildgebungsverfahren (100) nach Anspruch 1, wobei der Zielbereich mehrere Unterzielbereiche umfasst, die entlang der ersten Richtung (y) angeordnet sind, und wobei das Verfahren (100) das parallele Durchführen der folgenden Vorgänge auf jedem der mehreren Unterzielbereiche umfasst:
Beleuchten des Unterzielbereichs auf die Weise des Reihe-für-Reihe-Abtastens entlang der einzelnen ersten Richtung (y) bei gleichzeitigem Schalten der mehreren Typen von strukturiertem Zeilenanregungslicht, derart dass jede Reihe in dem Unterzielbereich mit einem entsprechenden Typ von strukturiertem Zeilenanregungslicht von den mehreren Typen von strukturiertem Zeilenanregungslicht beleuchtet wird; und
Detektieren von Reaktionslicht, das von jeder Reihe in dem Unterzielbereich als Reaktion darauf erzeugt wird, dass sie mit dem entsprechenden Typ von strukturiertem Zeilenanregungslicht beleuchtet wird.

12. Hochauflösende Bildgebungseinrichtung (200) auf Basis von Zeilenabtastung, umfassend:
ein Beleuchtungsmodul (220), das so ausgestaltet ist, dass es strukturiertes Zeilenanregungslicht bereitstellt;
ein Abtastmodul (240) das so ausgestaltet ist, dass es einen Zielbereich auf eine Weise eines Reihe-für-Reihe-Abtastens entlang einer einzelnen ersten Richtung (y) mit dem strukturierten Zeilenanregungslicht beleuchtet; und
ein Detektionsmodul (260), das so ausgestaltet ist, dass es Reaktionslicht detektiert, das von jeder Reihe in dem Zielbereich als Reaktion darauf erzeugt wird, dass sie mit dem strukturierten Zeilenanregungslicht beleuchtet wird,
**dadurch gekennzeichnet, dass**:
das Beleuchtungsmodul (220) so ausgestaltet ist, dass es mehrere Typen von strukturiertem Zeilenanregungslicht bereitstellt, wobei jeder der mehreren Typen von strukturiertem Zeilenanregungslicht ein unterschiedliches räumliches Verteilungsmuster der optischen Intensität aufweist,
das Abtastmodul (240) ferner so ausgestaltet ist, dass es die mehreren Typen von strukturiertem Zeilenanregungslicht während des Reihe-für-Reihe-Abtastens so schaltet, dass jede Reihe im Zielbereich mit einem entsprechenden Typ von strukturiertem Zeilenanregungslicht von den mehreren Typen von strukturiertem Zeilenanregungslicht beleuchtet wird, und
das detektierte Reaktionslicht von jeder Reihe im Zielbereich als Reaktion darauf erzeugt wird, dass sie mit dem entsprechenden Typ von strukturiertem Zeilenanregungslicht beleuchtet wird.

13. Hochauflösende Bildgebungseinrichtung (200) nach Anspruch 12, wobei die mehreren Typen von strukturiertem Zeilenanregungslicht erstes strukturiertes Zeilenanregungslicht und zweites strukturiertes Zeilenanregungslicht umfassen, wobei das erste strukturierte Zeilenanregungslicht einen Lichtfleck aufweist, der sich ununterbrochen in einer zweiten Richtung (x) senkrecht zur ersten Richtung (y) erstreckt, und das zweite strukturierte Zeilenanregungslicht eine Vielzahl von Lichtflecken aufweist, die in im Wesentlichen periodischen Intervallen in der zweiten Richtung (x) angeordnet sind,
wobei die im Wesentlichen periodischen Intervalle Intervalle mit einer Änderung von einer vorgegebenen Intervallperiode oder einer Zielintervallperiode sind, die in einem Bereich von ± 20 % der vorgegebenen Intervallperiode oder der Zielintervallperiode liegt, und
wobei das Abtastmodul (240) ferner so ausgestaltet ist, dass es während des Reihe-für-Reihe-Abtastens bewirkt, dass das zweite strukturierte Zeilenanregungslicht jedes Mal, wenn es zur Beleuchtung verwendet wird, im Vergleich zu seiner letzten Verwendung zur Beleuchtung, einer voreingestellten Phasenverschiebung in der zweiten Richtung (x) mit einem Phasenverschiebungsabstand unterzogen wird, der ein nicht ganzzahliges Vielfaches einer Intervallperiode der Lichtflecken des zweiten strukturierten Zeilenanregungslichts ist.

14. Hochauflösende Bildgebungseinrichtung (200) nach Anspruch 12, wobei das Beleuchtungsmodul (220) umfasst:
eine Lichtquelle (221), die dazu ausgestaltet ist, Anregungslicht bereitzustellen;
eine optische Aufteilungseinheit (222), die so ausgestaltet ist, dass sie das Anregungslicht von der Lichtquelle (221) in mehrere Strahlen von Anregungslicht aufteilt;
eine Vielzahl von Erzeugungseinheiten für strukturiertes Zeilenanregungslicht (2241, 2242, ..., 224N), wobei jede der Vielzahl von Erzeugungseinheiten für strukturiertes Zeilenanregungslicht (2241, 2242, ..., 224N) so ausgestaltet ist, dass sie einen entsprechenden Strahl von Anregungslicht von der optischen Aufteilungseinheit (222) empfängt und einen entsprechenden Typ von strukturiertem Zeilenanregungslicht basierend auf dem empfangenen entsprechenden Strahl von Anregungslicht erzeugt;
eine optische Kombinationseinheit (225), die so ausgestaltet ist, dass sie mehrere Typen von strukturiertem Zeilenanregungslicht von der Vielzahl von Erzeugungseinheiten für strukturiertes Zeilenanregungslicht (2241, 2242, ..., 224N) in einen Strahl kombiniert; und
eine Vielzahl von optischen Schalteinheiten (2231, 2232, ..., 223N), wobei jede der Vielzahl von optischen Schalteinheiten (2231, 2232, ..., 223N) zwischen der optischen Aufteilungseinheit (222) und einer entsprechenden der Erzeugungseinheiten für strukturiertes Zeilenanregungslicht (2241, 2242, ..., 224N) angeordnet ist und so ausgestaltet ist, dass sie steuert, ob das Anregungslicht von der optischen Aufteilungseinheit (222) an die entsprechende der Erzeugungseinheiten für strukturiertes Zeilenanregungslicht (2241, 2242, ..., 224N) ausgegeben wird, oder jede der Vielzahl von optischen Schalteinheiten (2231, 2232, ..., 223N) zwischen einer entsprechenden der Erzeugungseinheiten für strukturiertes Zeilenanregungslicht (2241, 2242, ..., 224N) und der optischen Kombinationseinheit (225) angeordnet ist und so ausgestaltet ist, dass sie steuert, ob das strukturierte Zeilenanregungslicht von der entsprechenden von den Erzeugungseinheiten für strukturiertes Zeilenanregungslicht (2241, 2242, ..., 224N) an die optische Kombinationseinheit (225) ausgegeben wird,
wobei das strukturierte Zeilenanregungslicht, das von unterschiedlichen Erzeugungseinheiten für strukturiertes Zeilenanregungslicht (2241, 2242, ..., 224N) von der Vielzahl von Erzeugungseinheiten für strukturiertes Zeilenanregungslicht (2241, 2242, ..., 224N) erzeugt wird, unterschiedliche räumliche Verteilungsmuster der optischen Intensität aufweist.

15. Hochauflösende Bildgebungseinrichtung (200) nach Anspruch 12, wobei das Beleuchtungsmodul umfasst:
eine Lichtquelle (221), die so ausgestaltet ist, dass sie Anregungslicht bereitstellt; und
eine einzelne Modulationseinheit (226), die so ausgestaltet ist, dass sie das Anregungslicht von der Lichtquelle (221) in strukturiertes Zeilenanregungslicht mit unterschiedlichen räumlichen Verteilungsmustern der optischen Intensität moduliert.

## Revendications

1. Procédé d'imagerie à super-résolution (100) basé sur un balayage de lignes, comportant :
la fourniture (S102) d'une lumière d'excitation à lignes structurées ;
l'éclairage (S104), par la lumière d'excitation à lignes structurées, d'une zone cible à la manière d'un balayage ligne à ligne le long d'une première direction (y) unique ; et
la détection (S106) d'une lumière de réponse générée par chaque ligne dans la zone cible en réponse au fait qu'elles sont éclairées par la lumière d'excitation à lignes structurées,
**caractérisé en ce que** :
de multiples types de lumière d'excitation à lignes structurées sont fournis, et chacun des multiples types de lumière d'excitation à lignes structurées a un motif de distribution spatiale d'intensité optique différent,
le procédé (100) comporte en outre la commutation des multiples types de lumière d'excitation à lignes structurées pendant le balayage ligne à ligne, de sorte que chaque ligne dans la zone cible soit éclairée par un type correspondant de lumière d'excitation à lignes structurées parmi les multiples types de lumière d'excitation à lignes structurées et
la lumière de réponse détectée est générée par chacune des lignes dans la zone cible en réponse au fait qu'elles sont éclairées par le type correspondant de lumière d'excitation à lignes structurées.

2. Procédé d'imagerie à super-résolution (100) selon la revendication 1, dans lequel les multiples types de lumière d'excitation à lignes structurées comportent une première lumière d'excitation à lignes structurées et une deuxième lumière d'excitation à lignes structurées, la première lumière d'excitation à lignes structurées a un point s'étendant en continu dans une seconde direction (x) perpendiculaire à la première direction (y) et la deuxième lumière d'excitation à lignes structurées a une pluralité de points agencés à des intervalles sensiblement périodiques dans la seconde direction (x),
dans lequel les intervalles sensiblement périodiques sont des intervalles avec un changement par rapport à une période d'intervalle de conception ou une période d'intervalle cible qui se trouve dans une plage de ±20 % de la période d'intervalle de conception ou de la période d'intervalle cible.

3. Procédé d'imagerie à super-résolution (100) selon la revendication 1 ou 2, dans lequel les multiples types de lumière d'excitation à lignes structurées comprennent de multiples types de deuxième lumière d'excitation à lignes structurées qui a une pluralité de points agencés à des intervalles sensiblement périodiques dans une seconde direction (x) perpendiculaire à la première direction (y) et chacun des multiples types de deuxième lumière d'excitation à lignes structurées a une combinaison de forme de point et de période d'intervalle différente de celle d'autres types parmi les multiples types de deuxième lumière d'excitation à lignes structurées,
dans lequel les intervalles sensiblement périodiques sont des intervalles avec un changement par rapport à une période d'intervalle de conception ou à une période d'intervalle cible qui se trouve dans une plage de ±20 % de la période d'intervalle de conception ou de la période d'intervalle cible.

4. Procédé d'imagerie à super-résolution (100) selon la revendication 2, dans lequel, pendant le balayage ligne à ligne, la deuxième lumière d'excitation à lignes structurées est soumise à un déphasage prédéfini dans la seconde direction (x) à chaque fois qu'elle est utilisée pour un éclairage par rapport à la dernière fois où elle a été utilisée pour un éclairage, avec une distance de déphasage qui est un multiple non entier d'une période d'intervalle des points de la deuxième lumière d'excitation à lignes structurées.

5. Procédé d'imagerie à super-résolution (100) selon la revendication 1, dans lequel les multiples types de lumière d'excitation à lignes structurées comprennent une troisième lumière d'excitation à lignes structurées et une quatrième lumière d'excitation à lignes structurées, un motif de distribution spatiale d'intensité optique de la troisième lumière d'excitation à lignes structurées est configuré pour améliorer les performances d'imagerie dans une troisième direction et un motif de distribution spatiale d'intensité optique de la quatrième lumière d'excitation à lignes structurées est configuré pour améliorer les performances d'imagerie dans une quatrième direction différente de la troisième direction.

6. Procédé d'imagerie à super-résolution (100) selon la revendication 5, dans lequel la troisième direction est parallèle à la première direction (y) et la quatrième direction est perpendiculaire à la troisième direction.

7. Procédé d'imagerie à super-résolution (100) selon la revendication 6, dans lequel le motif de distribution spatiale d'intensité optique de la quatrième lumière d'excitation à lignes structurées est configuré pour améliorer les performances d'imagerie à la fois dans la quatrième direction et dans une cinquième direction qui est perpendiculaire à la fois à la troisième direction et à la quatrième direction.

8. Procédé d'imagerie à super-résolution (100) selon la revendication 6, dans lequel les multiples types de lumière d'excitation à lignes structurées comportent en outre une cinquième lumière d'excitation à lignes structurées et un motif de distribution spatiale d'intensité optique de la cinquième lumière d'excitation à lignes structurées est configuré pour améliorer les performances d'imagerie dans une cinquième direction qui est perpendiculaire à la fois à la troisième direction et à la quatrième direction.

9. Procédé d'imagerie à super-résolution (100) selon la revendication 1, dans lequel les multiples types de lumière d'excitation à lignes structurées sont commutés selon un ordre prédéfini afin d'éclairer des lignes respectives dans la zone cible.

10. Procédé d'imagerie à super-résolution (100) selon la revendication 1, dans lequel, pendant le balayage ligne à ligne, des régions éclairées par deux applications consécutives de lumière d'excitation à lignes structurées dans la zone cible se chevauchent partiellement les unes les autres.

11. Procédé d'imagerie à super-résolution (100) selon la revendication 1, dans lequel la zone cible comporte de multiples sous-zones cibles agencées le long de la première direction (y) et dans lequel le procédé (100) comporte la réalisation des opérations suivantes sur chacune des multiples sous-zones cibles en parallèle :
l'éclairage de la sous-zone cible à la manière d'un balayage ligne à ligne le long de la première direction (y) unique tout en commutant les multiples types de lumière d'excitation à lignes structurées, de sorte que chaque ligne dans la sous-zone cible soit éclairée par un type correspondant de lumière d'excitation à lignes structurées parmi les multiples types de lumière d'excitation à lignes structurées ; et
la détection de lumière de réponse générée par chacune des lignes dans la sous-zone cible en réponse au fait qu'elles sont éclairées par le type correspondant de lumière d'excitation à lignes structurées.

12. Appareil d'imagerie à super-résolution (200) basé sur un balayage de lignes, comportant :
un module d'éclairage (220) configuré pour fournir une lumière d'excitation à lignes structurées ;
un module de balayage (240) configuré pour éclairer, par la lumière d'excitation à lignes structurées, une zone cible à la manière d'un balayage ligne à ligne le long d'une première direction (y) unique ; et
un module de détection (260) configuré pour détecter une lumière de réponse générée par chaque ligne dans la zone cible en réponse au fait qu'elles sont éclairées par la lumière d'excitation à lignes structurées,
**caractérisé en ce que** :
le module d'éclairage (220) est configuré pour fournir de multiples types de lumière d'excitation à lignes structurées, chacun des multiples types de lumière d'excitation à lignes structurées ayant un motif de distribution spatiale d'intensité optique différent,
le module de balayage (240) est en outre configuré pour commuter les multiples types de lumière d'excitation à lignes structurées pendant le balayage ligne à ligne, de sorte que chaque ligne dans la zone cible soit éclairée par un type correspondant de lumière d'excitation à lignes structurées parmi les multiples types de lumière d'excitation à lignes structurées et
la lumière de réponse détectée est générée par chacune des lignes dans la zone cible en réponse au fait qu'elles sont éclairées par le type correspondant de lumière d'excitation à lignes structurées.

13. Appareil d'imagerie à super-résolution (200) selon la revendication 12, dans lequel les multiples types de lumière d'excitation à lignes structurées comportent une première lumière d'excitation à lignes structurées et une deuxième lumière d'excitation à lignes structurées, la première lumière d'excitation à lignes structurées a un point s'étendant en continu dans une seconde direction (x) perpendiculaire à la première direction (y) et la deuxième lumière d'excitation à lignes structurées a une pluralité de points agencés à des intervalles sensiblement périodiques dans la seconde direction (x),
dans lequel les intervalles sensiblement périodiques sont des intervalles avec un changement par rapport à une période d'intervalle de conception ou à une période d'intervalle cible qui se trouve dans une plage de ±20 % de la période d'intervalle de conception ou de la période d'intervalle cible et
dans lequel le module de balayage (240) est en outre configuré pour, pendant le balayage ligne à ligne, amener la deuxième lumière d'excitation à lignes structurées à se soumettre à un déphasage prédéfini dans la seconde direction (x) à chaque fois qu'elle est utilisée pour l'éclairage par rapport à la dernière fois où elle a été utilisée pour l'éclairage, avec une distance de déphasage qui est un multiple non entier d'une période d'intervalle des points de la deuxième lumière d'excitation à lignes structurées.

14. Appareil d'imagerie à super-résolution (200) selon la revendication 12, dans lequel le module d'éclairage (220) comporte :
une source de lumière (221) configurée pour fournir une lumière d'excitation ;
une unité de division optique (222) configurée pour diviser la lumière d'excitation provenant de la source de lumière (221) en de multiples faisceaux de lumière d'excitation ;
une pluralité d'unités de génération de lumière d'excitation à lignes structurées (2241, 2242, ..., 224N), chaque unité parmi la pluralité d'unités de génération de lumière d'excitation à lignes structurées (2241, 2242, ..., 224N) étant configurée pour recevoir un faisceau correspondant de lumière d'excitation provenant de l'unité de division optique (222) et pour générer un type correspondant de lumière d'excitation à lignes structurées sur la base du faisceau de lumière d'excitation correspondant reçu ;
une unité de combinaison optique (225) configurée pour combiner de multiples types de lumière d'excitation à lignes structurées provenant de la pluralité d'unités de génération de lumière d'excitation à lignes structurées (2241, 2242, ..., 224N) en un faisceau ; et
une pluralité d'unités de commutation optique (2231, 2232, ..., 223N), chaque unité parmi la pluralité d'unités de commutation optique (2231, 2232, ..., 223N) étant disposée entre l'unité de division optique (222) et une unité correspondante parmi les unités de génération de lumière d'excitation à lignes structurées (2241, 2242, ..., 224N) et étant configurée pour commander le fait que la lumière d'excitation provenant de l'unité de division optique (222) soit ou non sortie vers l'unité correspondante parmi les unités de génération de lumière d'excitation à lignes structurées (2241, 2242, ..., 224N) ou chaque unité parmi la pluralité d'unités de commutation optique (2231, 2232, ..., 223N) étant disposée entre une unité correspondante parmi les unités de génération de lumière d'excitation à lignes structurées (2241, 2242, ..., 224N) et l'unité de combinaison optique (225) et étant configurée pour commander le fait que la lumière d'excitation à lignes structurées provenant de l'unité correspondante parmi les unités de génération de lumière d'excitation à lignes structurées (2241, 2242, ..., 224N) soit ou non sortie vers l'unité de combinaison optique (225),
dans lequel la lumière d'excitation à lignes structurées générée par différentes unités de génération de lumière d'excitation à lignes structurées (2241, 2242, ..., 224N) parmi la pluralité d'unités de génération de lumière d'excitation à lignes structurées (2241, 2242, ..., 224N) a différents motifs de distribution spatiale d'intensité optique.

15. Appareil d'imagerie à super-résolution (200) selon la revendication 12, dans lequel le module d'éclairage comporte :
une source de lumière (221) configurée pour fournir une lumière d'excitation ; et
une unité de modulation (226) unique configurée pour moduler la lumière d'excitation provenant de la source de lumière (221) en une lumière d'excitation à lignes structurées avec différents motifs de distribution spatiale d'intensité optique.
